# EUROPEAN PATENT APPLICATION

(11) **EP 3 988 645 A1**
(43) Date of publication of application: **27.04.2022**
(21) Application number: 20827930.7
(22) Date of filing: 19.05.2020
(51) Int. Cl.: C12N 5/00, C12N 5/077, C12N 5/071, A61L 27/36

(54) **DECELLULARIZATION METHOD**

(30) Priority: 21.06.2019 KR 20190074334
(71) Applicant: S&G Biotech, Inc., Yongin-si, Gyeonggi-do 17023 (KR)
(72) Inventor: LAKTIONOV, Pavel Petrovich, Novosibirsk, 630090 (RU); KARPENKO, Andrey Anatol'evich, Novosibirsk, 630058 (RU); CHERNONOSOVA, Vera Sergeevna, Novosibirsk, 630117 (RU); CHEREPANOVA, Anna Vital'evna, Novosibirsk, 630055 (RU); RASSKAZOV, Georgy Alexandrovich, Novosibirsk region, 633003 (RU)
(74) Representative: Shearman, James Ward
(86) International application number: PCT/KR2020/006514
(87) International publication number: WO 2020/256286

(57) **Abstract**

The purpose of the present invention is to provide: detergent-free decellularization method of xenogenic biological tissues for human body surgery, in which the pericardium, blood vessels, other membrane-like biological tissues, and the like are decellularized so as to have resistance to mechanical property loss, mineralization and immune reactivity; and decellularized tissue. Decellularized tissue, according to the present invention, when compared to untreated tissue, has greater calcification reduction in vivo, blood compatibility and biocompatibility improvement, tissue thickness reduction, and increases in tensile strength, kink resistance and the like.

## Description

### TECHNICAL FIELD

The present invention relates to a tissue decellularization method, and in particular, to a method for treating relatively thin flat or tubular non-parenchymal tissues, such as the pericardium, small intestine, blood vessels, and dermis, to have acellular and non-immunogenic properties, to be filled with matrix proteins, such as collagen and elastin, and to have high density, abrasion resistance, high rigidity, excellent bio/blood compatibility, and superior resistance to mineralization and exfoliation, and a tissue decellularized by the same.

### BACKGROUND ART

Atherosclerosis is the most common disease in humans leading to ischemic tissue lesions, and its treatment requires replacement of cardiovascular system components, such as arteries, veins, and heart valves. Meanwhile, this is addressed by replacing the valve with a mechanical or bioprosthetic valve. Bioprosthetic valves typically consist of lobules and vascular conduits or, if used for transcatheter transfer, of lobules and skirts. Bioprosthetic valves have many advantages over mechanical valves, including a lower risk of complications from thrombus formation but may often have some issues, such as loss of mechanical properties and calcification (mineralization). Calcification is a limiting factor critical in the life expectancy of the currently used bioprosthetic heart valves, which may reduce the thickening, contraction and migration of lobules and cause stenosis. Since typical treatment for functionally damaged prosthetic valves is replacement with new valves, the short useful life of the prosthetic heart valve is a serious medical problem for patients and financial loss of the medical system. Aneurysms may also occur due to loss of mechanical load of decellularized artificial blood vessels. Addressing this is an important challenge, as these properties significantly limit the durability of replacement valves and blood vessels.

In relation thereto, Patent Document 1 proposes a method for decellularizing a living tissue by exposing it to a buffer solution. However, such a decellularization method also suffers from loss of mechanical properties and calcification.

### [PRIOR TECHNICAL DOCUMENTS]

### [PATENT DOCUMENTS]

(Patent Document 1) U.S. Patent No. 5,595,571

### [Non-Patent literature]

(Non-Patent Document 1) Leesson-Dietrich et al, J. Heart Valve Disease 4:88 (1995)
(Non-Patent Document 2) Chernonosova VS, 2018
(Non-Patent Document 3) Theory and Practice of Histological Techniques, edited by Bancroft and Stephens, Churchill Livingstone, Edinburgh (1990)

### DETAILED DESCRIPTION OF THE INVENTION

### TECHNICAL PROBLEMS

The present invention aims to provide a method for decellularizing the pericardium, blood vessels, or other membrane-like biological tissues, and a decellularized tissue, thus preventing loss of mechanical properties of biological tissues and mineralization and allowing the living tissue to have resistance to immune reactivity.

According to the present invention, the decellularized tissue is intended to be used as a substitute for defective tissue in mammals, particularly humans. For example, one of ordinary skill in the art may use it to replace heart valves, blood vessels, and the like by known techniques.

### MEANS TO ADDRESS THE PROBLEMS

The present invention provides a method for preparing a material for treating a mammal by replacing a defective living tissue, wherein the defective tissue is selected from among a heart valve, an artery, a vein, a diaphragm, a pericardium, a fascia, a dura mater or an eardrum, and the material contains a decellularized and fixed living tissue. Specifically, the present invention provides a method for preparing a decellularized tissue comprising the following steps and a tissue prepared thereby:
(i) obtaining, from a mammal, a tissue selected from a heart valve, artery, vein, diaphragm, pericardium, fascia, dura mater or tympanic membrane;
(ii) washing the obtained tissue with a washing solution;
(iii) placing the washed tissue in a transmembrane liquid flow device;
(iv) washing the tissue with a buffer solution under a pressure of 0.3 to 2 MPa until A ₂₆₀ optical density is less than 0.1;
(v) washing the tissue with water under a pressure of 0.3 to 2 MPa until the A ₂₆₀ optical density is less than 0.05;
(vi) washing the tissue with a solution containing a bifunctional compound under a pressure of 0.3 to 2 MPa;
(vii) after removal of the tissue from the transmembrane fluid flow device, immobilizing the tissue under a pressure of 2 to 10 kg/cm²; and
(viii) washing the tissue.

### EFFECTS OF THE INVENTION

The tissue decellularized according to the present invention have reduced mineralization (e.g., calcification) in vivo as compared to untreated tissues. Further, the decellularization according to the present invention reduces the immunogenicity of the tissue and enhances blood compatibility and biocompatibility. Further, the method reduces the thickness of the tissue and increases the tensile strength and twist resistance.

The tissue decellularized according to the present invention decreases in damage to the extracellular matrix and loss of collagen, elastin and glycosaminoglycan (GAG) as compared to the tissue decellularized using detergent.

### BRIEF DESCRIPTION OF THE DRAWINGS

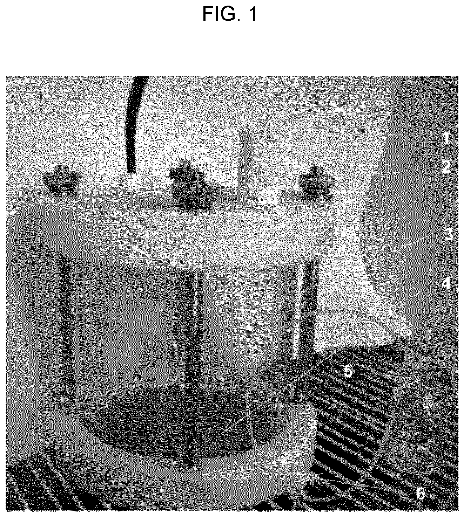

FIG. 1 illustrates a transmembrane liquid flow device of a living tissue. Specifically, in FIG. 1, 1 denotes a safety valve; 2 denotes an additional pressure input device; 3 denotes a liquid storage container under overpressure; 4 denotes a porous support for installing a living tissue; 5 denotes a collector of the fluid that has passed through the pericardium; 6 denotes an outlet of the liquid.

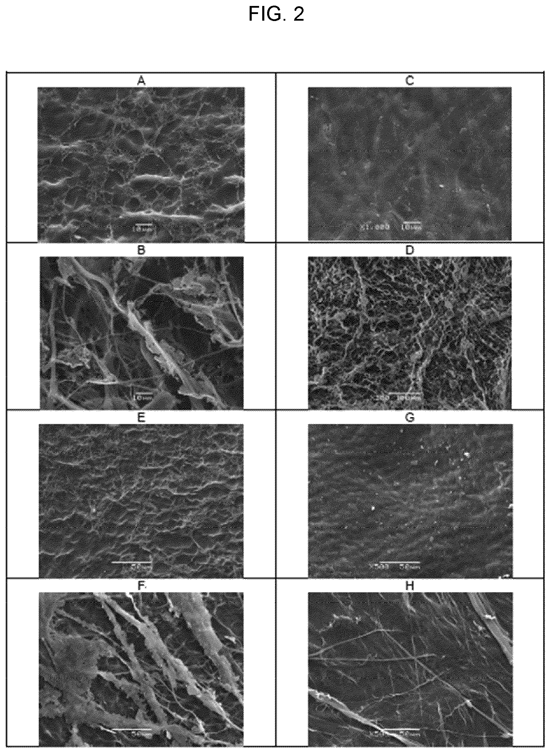

FIG. 2 illustrates the results of electron microscopy observation of the effect of decellularization treatment on the surface structure of the porcine pericardium. Specifically, FIG. 2A illustrates pericardium 1 before treatment; FIG. 2B illustrates the opposite side of pericardium 1 before treatment; FIG. 2C illustrates pericardium 1 after treatment; FIG. 2D illustrates the opposite side of pericardium 1 after treatment; FIG. 2E illustrates pericardium 2 before treatment; FIG. 2F illustrates the opposite side of pericardium 2 before treatment; FIG. 2G illustrates pericardium 2 after treatment with Folch reagent; and FIG. 2H illustrates the opposite side of pericardium 2 after treatment with Folch reagent.

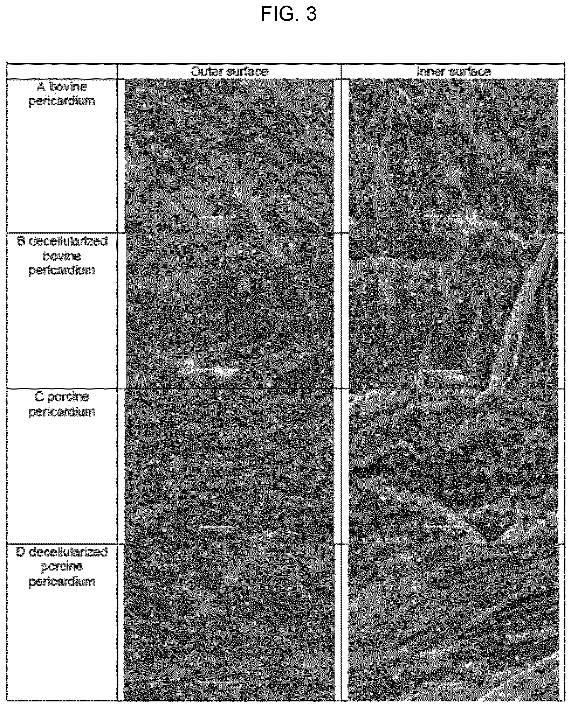

FIG. 3 illustrates the results of electron microscopy (SEM) observation of the effect of decellularization on the outer and inner surfaces of bovine and porcine pericardium. Specifically, FIG. 3A illustrates the bovine pericardium before treatment, FIG. 3B illustrates the bovine pericardium after treatment, FIG. 3C illustrates the porcine pericardium before treatment, and FIG. 3D illustrates the porcine pericardium after treatment.

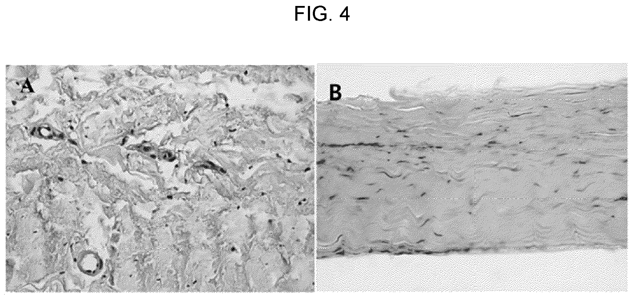

FIG. 4 illustrates the results of histological observation of bovine pericardium before decellularization (A) and after decellularization (B). A tissue was stained with hematoxylin and observed at 400 magnification.

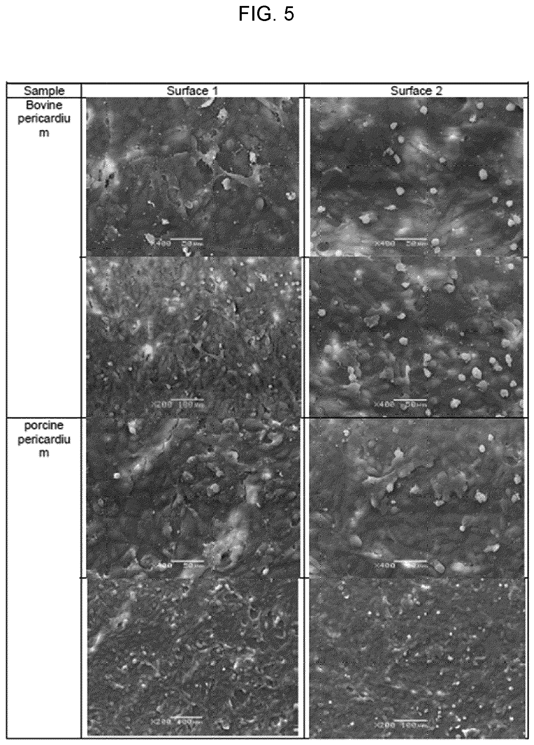

FIG. 5 illustrates the results of culturing human umbilical vein endothelial cells (HUVECs) in decellularized bovine and porcine pericardium.

### BEST MODE TO PRACTICE THE INVENTION

Hereinafter, the present invention is described in greater detail.

The present invention relates to a method for preparing a decellularized tissue comprising the steps of:
(i) obtaining, from a mammal, a tissue selected from a heart valve, artery, vein, diaphragm, pericardium, fascia, dura mater or tympanic membrane;
(ii) washing the obtained tissue with a washing solution;
(iii) placing the washed tissue in a transmembrane liquid flow device;
(iv) washing the tissue with a buffer solution under a pressure of 0.3 to 2 MPa until A ₂₆₀ optical density is less than 0.1;
(v) washing the tissue with water under a pressure of 0.3 to 2 MPa until the A ₂₆₀ optical density is less than 0.05;
(vi) washing the tissue with a solution containing a difunctional compound under a pressure of 0.3 to 2 MPa;
(vii) after removal of the tissue from the transmembrane fluid flow device, immobilizing the tissue under a pressure of 2 to 10 kg/cm²; and
(viii) washing the tissue.

In the present invention, decellularization is a process for removing cells in a living tissue. Preferably, the tissue may be essentially decellularized. Here, the term "essentially" means that the tissue has at least 95% fewer cells than a naturally occurring living tissue. In determining essential decellularization, only cells unique to the living tissue are counted. Blood-mediated cells, including red blood cells and platelets, as well as cells of other organisms, are not included.

In the present invention, tissue refers to a living tissue suitable for transplantation into humans or animals. The tissue may be from a human or non-human animal (e.g., bovine, porcine tissue or others). Preferably, the tissue may be bovine or porcine tissue. Preferably, fresh tissue is preferred. The tissue may be cryopreserved but, in such a case, its mechanical properties after decellularization may be different from those of fresh tissue. Although the present invention takes the pericardium as an example, the decellularization method is also applicable to other tissues including heart valves, fascia, arteries, veins, diaphragm, umbilical cord, mesentery, dura mater, or tympanic membrane.

In step (i), the tissue is obtained from a source known to one of ordinary skill in the art. For example, tissue may be obtained from animals obtained from slaughterhouses that are subjected to examination to ensure the provision of living tissue of adequate quality. The living tissue does not necessarily need to be obtained under aseptic conditions, but is preferably obtained under clean conditions to reduce contamination. After obtaining the living tissue, the tissue may be stored in a buffer solution having a temperature range of about 3 to 10 °C. The pH range of the solution may be about 7.0 to 7.8. The temperature and pH of the solution may be selected to preserve the fresh state of the living tissue. Thus, for certain buffer solutions and conditions, pH and temperature may fall outside the above ranges. The living tissue may be stored for a long time ranging from days to weeks. Preferably, the next step may commence on the living tissue within hours of harvesting for maximum preservation of the structural components of the tissue. More preferably, the next step may begin on the tissue within 2 to 10 hours after harvesting.

The washing in step (ii) is a process of treating with a washing solution to remove the blood components attached to the surface of the living tissue, reduce the amount of contaminants, and keep the tissue fresh. In the present invention, the washing solution may be various washing solutions known to one of ordinary skill in the art, and preferably, the washing solution is a buffer solution. More preferably, the washing solution is PBS. Further, the washing solution may include a bacteriostatic agent, and a bacteriostatic agent known to one of ordinary skill in the art may be used. Washing may be performed at a temperature of about 0 to 30°C until all traces of blood cells are removed. Preferably, washing is carried out at a temperature of about 0 to 20 °C. Washing may be done with, e.g., PBS under aseptic conditions with a bacteriostatic agent. The next step may be started immediately after washing or within one day.

The present invention may further comprise, after step (ii), washing the tissue obtained in step (ii) with an isotonic solution containing DNAase or RNAase, preferably PBS, to further remove DNA and RNA from the tissue.

The transmembrane liquid flow device in step (iii) is illustrated in FIG. 1 as a device for transmembrane flow of liquid or device for cross flow for decellularization, and it typically includes a support with a through hole, an O-ring, a liquid storage tank capable of operating under pressure, a safety valve, and accessories. In the transmembrane liquid flow device, the tissue placed on the porous support is put, and is passed through by the liquid in a cross flow, so that the liquid reaches uniformly the thickness of the tissue. With an appropriate choice of liquid, the tissue may be filled with polysaccharides, proteins, anticoagulants, and the like.

Washing in step (iv) is a process of washing with a buffer solution until A ₂₆₀ optical density is less than 0.1 to thereby remove the aqueous tissue and serum proteins, such as annexins, albumins, and immunoglobulins, and lyse some cells by pressure stress. The buffer solution may be any of a variety of buffer solutions known to one of ordinary skill in the art. The buffer solution may include a variety of phosphate and non-phosphate buffer solutions. Preferred buffer solutions include phosphate buffer solutions, such as sodium phosphate monobasic and dibasic buffer solutions and phosphate citrate buffer solutions. More preferably, the buffer solution is PBS. The buffer solution may be supplied by 5 to 10ml per 10cm² of the tissue depending on the thickness of the tissue, and a pressure of 0.3 to 2 MPa may be applied depending on the tissue thickness and porosity/density until the entire buffer solution passes through the tissue. This step may be carried out at a temperature of 0 to 37 °C, preferably at a temperature of 2 to 6 °C, typically for 5 to 24 hours in a nitrogen atmosphere. After step (iv) is completed, the pressure is relieved.

Washing in step (v) is a process of completely lysing the cells through hypotonic treatment and removing biopolymers and organelles by washing the tissue with water until A ₂₆₀ optical density is less than 0.05. Preferably, the water may be pyrogen-free water. The water may be supplied by 5 to 10ml per 10cm² of the tissue depending on the thickness of the tissue, and a pressure of 0.3 to 2 MPa may be applied depending on the tissue thickness and porosity/density until the entire water passes through the tissue. This step may be carried out at a temperature of 0 to 37 °C, preferably at a temperature of 2 to 6 °C, typically for 5 to 24 hours in a nitrogen atmosphere. After step (v) is completed, the pressure is relieved.

Measurement of A₂₆₀ optical density or absorbance may be performed through a known ultraviolet spectrometer in a scheme known to one of ordinary skill in the art.

The present invention may further comprise, after step (v), washing the tissue obtained in step (v) with a solution containing chloroform, water and ethanol, for fat removal from the tissue, reduction of calcification and reduction of occlusion/reduction of aldehyde/Schiff groups. Preferably, the solution contains 20 to 30% by mass of chloroform, 20 to 30% by mass of water, and 45 to 50% by mass of ethanol. More preferably, the solution is a Folch solution containing 25% by mass of chloroform, 25% by mass of water, and 50% by mass of ethanol.

Washing in step (vi) is a process for inducing a crosslinking reaction by washing the tissue with a solution containing a difunctional compound. The difunctional compound may be a compound that acts as a cross-linking agent, preferably a bis-epoxy compound, and more preferably, polyethylene glycol-diglycidyl ether (PEG-DGE) or glutaraldehyde (GA). The solution containing the difunctional compound may contain 0.5 to 2% by mass of the difunctional compound. Further, in step (vi), the solution containing the difunctional compound may be treated in a volume ratio of 10 to 200x, e.g., in a volume ratio of 100x. The buffer solution containing the difunctional compound may be supplied by 5 to 10ml per 10cm² of the tissue depending on the thickness of the tissue, and a pressure of 0.3 to 2 MPa may be applied depending on the tissue thickness and porosity/density until the entire solution passes through the tissue. This step may be carried out at a temperature of 0 to 37 °C, preferably at a temperature of 2 to 6 °C, typically for 5 to 24 hours in a nitrogen atmosphere. After step (vi) is completed, the pressure is relieved.

The immobilization in step (vii) is a process of fixing the living tissue between two smooth surfaces, e.g., glass plates, and applying pressure to remove the pores which are the cause of the tissue degradation and mechanical property loss. Preferably, the pressure may be 2 to 10 kg/cm². The living tissue may be stored at a temperature of 4 to 25°C for 4 hours to 4 days depending on the type of the used difunctional compound and the tissue.

The present invention may further comprise, after step (vii), washing the tissue obtained in step (vii) with a solution containing a difunctional compound and protein/polysaccharide to enhance tissue bio/blood compatibility and preventing calcification. Preferably, the protein/polysaccharide is a solution containing human serum albumin, human fibronectin, laminin, vitronectin, collagen, human growth factor, hirudin, bivalirudin or heparin.

The present invention may further comprise, after step (vii), washing the tissue obtained in step (vii) with a solution containing an amino acid, followed by washing with a reducing agent. Preferably, the amino acid is glycine. Preferably, the reducing agent is sodium borohydride or sodium boro-cyano-hydride.

Washing in step (viii) is a process for washing the living tissue with a washing solution. For example, the washing solution in a volume ratio of 1000x or more may be exchanged 2 to 10 times, or the tissue may be washed with the washing solution in the transmembrane liquid flow device. As the washing solution, the washing solution described in step (ii) above may be used.

The present invention may further comprise the step of sterilizing. For example, sterilization may be performed in the presence or absence of a photosensitizer, by gamma or electron beam irradiation, ethylene oxide, b-propiolactone, povidone-iodine, or UV irradiation.

In the present invention, an agent for increasing blood compatibility, a platelet modulator, and an antibiotic may be additionally administered according to a method known to one of ordinary skill in the art.

Hereinafter, a specific method of the present invention is described in detail by way of embodiments, but the technical scope of the present invention is not limited to these embodiments.

### [Embodiment]

### Preparation Example 1

Phosphate-buffered saline (PBS) (Sigma, cat n. P4417-100TAB): 1 tablet was dissolved in pyrogen-free water as recommended.

1M CaCl₂: 2.22g of CaCl₂ was added to 20 ml of deionized water and stored at 4 °C.

1M MgCl₂: 2.033g of MgCI was added to 10 ml of deionized water and stored at 4 °C.

DNAse I solution: 10 mg DNAse I (Sigma D5025) (final concentration 0.2 mg/ml), 50 ul 1 M MgCl₂ (final concentration 5 mM), and 50 ul 1 M CaCl₂ (final concentration 5 mM) in 4.9 ml of sterilized water were dispensed into 1.5 ml microtubes marked for cooling, and stored refrigerated at -20 °C.

RNAse A solution: 100 mg of RNAse A was added to 10 ml of sterilized water, mixed, and dissolved, and 500 ml thereof was dispensed into each of 20 previously cooled 1.5 ml microtubes and stored at -20 °C.

Nuclease solution: 1 ml of 2 mg/ml DNAse I and 1 ml of 10 mg/ml RNAse A were added to 98 ml PBS, 1 M MgCl₂ (final concentration 3 mM), 96 ml 1 M CaCl₂ (final concentration 0.1 mM) and filter-sterilized with a 0.2 micron filter.

2% polyethylene glycol-diglycidyl ether (PEG-DGE) solution: 0.2 g polyethylene glycol-diglycidyl ether (Sigma) was mixed with 9.8 ml of pyrogen-free water.

0.25% glutaraldehyde (GA): 1 ml of 25% Ga solution (Sigma)

was mixed with 9 ml of pyrogen-free water.

Protein solution: was prepared at a concentration of 1 to 10 mg/ml and mixed with a difunctional solution immediately before use.

### decellularization of porcine pericardium

### Day 1

A porcine heart was obtained from a commercially available standard body weight pig (>120 kg). After rinsing in sterile PBS, the pericardium was removed and transported in sterile PBS at 4°C. All pericardium arrived within 6 hours of slaughter. PBS was replaced 5 or 6 times to remove remaining thrombus and blood cells remaining in the pericardium. A fresh pericardium (within 4-6 hours after removal) was placed on the porous support of the transmembrane liquid flow device for decellularization, and the remainder was cut out. The support with the pericardium was then installed in the device. The tank was filled with 15 to 10ml of PBS per 10cm² of the tissue depending on the thickness of the tissue. Then, a pressure of 0.3 to 2 MPa was applied depending on the tissue thickness and porosity/density until the entire PBS passed through the tissue. This process was performed at 4 °C until A ₂₆₀ optical density became less than 0.1.

### Day 2

After relieving the pressure, pyrogen-free water was added under the same conditions as PBS, and pressure was applied again until the entire water passed. Depending on the tissue thickness and porosity/density, a pressure of 0.3 to 2 MPa was applied until the entire PBS penetrated and permeated the tissue. This process was performed at 4 °C until A ₂₆₀ optical density became less than 0.05.

### Day 3

After relieving the pressure, a 2% PEG-DGE solution was added under the same conditions as PBS, and pressure was applied again until the entire solution passed. Depending on the tissue thickness and porosity/density, a pressure of 0.3 to 2 MPa was applied until the entire PBS penetrated and permeated the tissue. This process was carried out at 4 °C overnight.

### Days 4-7

The pericardium was straightened on a flat piece of glass and was covered with a second glass plate
and was pressed at 2 to 10 kg/cm². It was stored at a temperature of 4 to 6 °C for 4 days in a 100% humidity chamber.

### Days 8-9

To remove the excess reagent, the pericardium was cultured in excess PBS, which was replaced 4 times at 4°C under gentle agitation.

### Preparation Example 2

### solution preparation

The following solution was further used in the same solution as in Preparation Example 1.

Folch solution: 25 mass % of chloroform, 25 mass % of water, and 50 mass % of ethanol were mixed.

### decellularization of porcine pericardium

The step of washing the tissue with the Folch solution was added, after the step of the second day, to the same process as in Preparation Example 1.

### Embodiment 1

### Method for Measuring Calcium in Tissue Sample

Each recovered tissue was washed three times with 10 ml of sterile PBS free of calcium and magnesium. Calcium was quantified by X-ray photoelectron spectroscopy (XPS) at 30 points of the leaves and skirt of the detached plate. XPS analysis was performed with a SPECS electron spectrometer (SPECS GmbH, Germany) equipped with a PHOIBOS-150 MCD-9 hemispherical analyzer and a non-monochromatic MgK source. To prevent thermal degradation of the sample, the X-ray gun was positioned 30 mm away from the sample holder and operated only at 100 W. Spectra were recorded with pass energies set to have 50 eV (irradiation scan) and 10 eV (high resolution scan). Prior to the measurement, the energy scale was calibrated using Au4f_{7/2}(84.00 eV) and Cu2p_{3/2}(932.67 eV) peak of gold and copper thin films. The residual gas pressure while obtaining the spectrum was less than 3×10⁻⁷ Pa. The sample was mounted on a steel sample holder using 3M^{®} conductive copper double-sided adhesive tape. Data were processed quantitatively using XPSPEAK software version 4.1 and atomic sensitivity factors. The initial pericardium and the decellularized pericardium were compared, and the level of calcification was shown considering the control group.

### Biomechanical testing

Stiffness data were obtained using square-shaped pericardial samples. The thickness of each sample was obtained from the average of three measurements performed using a conductance circuit and a low-mass pin attached to a digital caliper. All tests were performed using wet samples at room temperature. Each sample was preconditioned to a load of 150 g until the continuous load-elongation curves overlapped (up to 20 cycles). Then, a uniaxial limiting tensile strength test for tissue degradation was performed. At least five specimens of each tissue type were examined.

Specifically, the stress-strain relationship was determined by the low-load elongation by applying a load of up to 150 g to the tissue at an elongation rate of 10 mm/min reflecting the study of leaflet biomechanics disclosed in Non-Patent Document 1.

### SEM and histochemistry

Immunohistochemical staining may be used to visualize cell-specific markers, such as smooth muscle actin and histocompatibility antigens, and the absence of these markers is an additional indicator of decellularization. Samples of fresh pericardium and decellularized pericardium were fixed with 2% formalin at 4°C. Pericardial fragments were dehydrated in ethanol solution series (50%, 70% and 96% ethanol) for scanning electron microscopy and then treated with hexamethyldisilazane (Sigma, USA). These fragments were fixed with conductive adhesive tape and sputter-coated with a 10 nm gold thin film and were examined using a JSM-6460 LV (Jeol, Japan) microscope as disclosed in Non-Patent Document 2. Paraffin slides were prepared and processed by methods known to one of ordinary skill in the art, and used for histological evaluation. The fragments with a thickness of 5 to 10 mm were stained with hematoxylin and eosin or stained by von Kossa (refer to Non-Patent Document 3) staining, with calcium specified.

### Statistics

Statistical differences in biomechanical parameter group average were assessed by independent t-tests. A p-value of 0.05 was selected as a significant difference value. Calcium data were analyzed according to ANOVA tests performed with statistical programs for IBM-PC and SPSS-PC.

### Results

### Substances lost during decellularization

DNA loss may be estimated by UV spectroscopy and biochemical methods using fluorescent dyes. Treatment of the pericardium with PBS immediately led to cell destruction and an increase in absorbance at 260 nm. Data on the DNA concentration obtained with the fluorescent dye Hoechst 33258 demonstrate that there are about 6000 cells per cm2 of the pericardial tissue The results are shown in Table 1 below.

### Mechanical properties of the pericardium

In the decellularized tissue, the pores remaining after cell lysis make the tissue more inhomogeneous and may cause tissue disintegration. Thus, the pericardium was rendered more uniform in structure and reduced in thickness by being fixed between two glasses at a pressure of 2 to 10kg/cm². This is a very important property for transcatheter transfer of heart valves and other applications. The tissue fixation process served to reduce the tissue thickness by up to 1.7 times together with cross-flow fluid passing through the pericardium (see Table 1). Decellularization increased the tissue tensile strength and suture retention rate and slightly decreased Young's modulus. For reference, Young's modulus means a coefficient indicating how the length of an elastic object changes relative to the deformation force.

### Biopsy

It was identified that decellularization resulted in complete loss of cells in the pericardium and compression of the structure but collagen fibers in the tissue were maintained.

Specifically, it was identified that porcine pericardium decellularized for one month was free of endogenous cells and sediment within the tissue matrix. The tissue calcium measured in this group was 60±14 mg/g, and calcareous sediments were found only in some parts. When further examination was performed using von Kossa stain, the corresponding part was clearly revealed. In particular, calcium sediments in the corresponding part appeared in non-specific structures. In contrast, early calcifications of glutaraldehyde-fixed tissues without decellularization were always present in the cell nucleus.

It was identified that as 1, 2, and 4 months passed after transplantation, the degree of inclusion of the leaf tissue in the calcified portion gradually increased. The center of the leaf was calcified earlier, and the edges were calcified later. Among the aortic or pulmonary valve vascular components, calcified portions generally remained at the periphery of the transplanted site, and mineralization was rarely observed in the central tissue of the transplanted site.

Table 1 below illustrates data on DNA and protein loss during decellularization and the thickness and density of the pericardium showing the effect of decellularization.

**[Table 1]**

| Preparation examples | Thickness, before/after, µm | * density g/cm²/square cm² | ** A260/280/ml | 260/280 ratio | Total DNA amount ng | Total protein amount µg | Tensile strength, before/after, mPa | Young's modulus, before/after |
|---|---|---|---|---|---|---|---|---|
| Preparation example 1 | 145±25/78±9 | 0.029±0.06/14±0.2 | 1.86±0.2/1.11±0.18 | 1.68±0.12 | 560±76 | 1760±0.215 | 8.5±0.7/12±1.5 | 3.5±0.3/5.2±0.5 |
| Preparation example 2 | 145±25/53±6 | 0.028±0.04/14±0.2 | 1.82±0.2/1.05±0.12 | 1.73±0.12 | 495±83 | 1650±0.28 | 7.6±0.7/10±1.1 | 3.0±0.4/4.8±0.6 |

| | | | | | | | | |
|---|---|---|---|---|---|---|---|---|
| * describe date of initial pericardium ** describe UV absorbance of entire PBS+H₂O | | | | | | | | |

### SEM

The results of electron microscopy observation of the effect of decellularization treatment on the surface structure of the porcine pericardium were shown in FIG. 2. Specifically, FIG. 2A illustrates pericardium 1 before treatment, FIG. 2B illustrates the opposite side of pericardium 1 before treatment, FIG. 2C illustrates pericardium 1 after treatment, FIG. 2D illustrates the opposite side of pericardium 1 after treatment, FIG. 2E illustrates pericardium 2 before treatment, FIG. 2F illustrates the opposite side of pericardium 2 before treatment, FIG. 2G illustrates pericardium 2 after treatment with Folch reagent, and FIG. 2H illustrates the opposite side of pericardium 2 after treatment with Folch reagent. Thus, it was identified that decellularization did not significantly affect the surface structure but made the inner surface of the tissue flatter.

### Embodiment 2

A porcine pericardium decellularized according to the method described in Preparation Example 1 and a bovine pericardium decellularized by the same method were prepared.

### Mechanical properties of the pericardium and SEM

A biomechanical test, SEM and histochemical test were performed in the same manner as described in embodiment 1. Specifically, the outer and inner surfaces of bovine and porcine pericardium before and after decellularization were observed with an electron microscope (SEM). As a result, as shown in FIG. 3, it was observed that the inner surface of the decellularized bovine and porcine pericardium was flattened compared to before the treatment, and the outer surface of the pericardium was hardly affected. Specifically, FIG. 3A illustrates the bovine pericardium before treatment, FIG. 3B illustrates the bovine pericardium after treatment, FIG. 3C illustrates the porcine pericardium before treatment, and FIG. 3D illustrates the porcine pericardium after treatment.

Further, the bovine pericardium before and after decellularization was stained with hematoxylin and histologically observed at 400x magnification. As a result, as shown in FIG. 4, no significant change was observed in the pericardial tissue before decellularization (A) and after decellularization (B). Specifically, there were observed no changes that would impede the function of the transplanted organ, such as tissue decomposition. Meanwhile, it was observed that the structure of the tissue became denser. Further, it was identified that the tissue was hardened, such as an increase in the tensile strength of the tissue due to decellularization.

### Cell culture experiment

Meanwhile, human umbilical vein endothelial cells (HUVECs) were cultured on the surface of the pericardium for cytotoxicity experiments in decellularized bovine and porcine pericardium. The cells were inoculated in the amount of 10,000 cells/cm² and cultured for 2 days in IM DMEM containing 10% fetal calf serum (FBS).

The results of the experiment are shown in FIG. 5. It was observed that continuous mono-layered cell layers were formed on both surfaces of the pericardium, and human umbilical vein endothelial cells were efficiently attached to the tissue and rapidly proliferated. In other words, it was identified that the decellularized tissue might be used as a transplantation organ in vivo because of no toxicity.

## Claims

1. A method for preparing a decellularized tissue, the method comprising the steps of:
(i) obtaining, from a mammal, a tissue selected from a heart valve, artery, vein, diaphragm, pericardium, fascia, dura mater or tympanic membrane;
(ii) washing the obtained tissue with a washing solution;
(iii) placing the washed tissue in a transmembrane liquid flow device;
(iv) washing the tissue with a buffer solution under a pressure of 0.3 to 2 MPa until A₂₆₀ optical density is less than 0.1;
(v) washing the tissue with water under a pressure of 0.3 to 2 MPa until the A₂₆₀ optical density is less than 0.05;
(vi) washing the tissue with a solution containing a difunctional compound under a pressure of 0.3 to 2 MPa;
(vii) after removal of the tissue from the transmembrane fluid flow device, immobilizing the tissue under a pressure of 2 to 10 kg/cm²; and
(viii) washing the tissue.

2. The method of claim 1, wherein the washing solution in step (ii) is PBS.

3. The method of claim 1, wherein the buffer solution in step (iv) is PBS.

4. The method of claim 1, further comprising, after step (ii), washing the tissue obtained in step (ii) with PBS containing DNAase or RNAase.

5. The method of claim 1, further comprising, after step (v), washing the tissue obtained in step (v) with a solution containing 25% by mass of chloroform, 25% by mass of water, and 50% by mass of ethanol.

6. The method of claim 1, further comprising, after step (vii), washing the tissue obtained in step (vii), a solution containing a difunctional compound; and a solution containing human serum albumin, human fibronectin, laminin, vitronectin, collagen, a human growth factor, hirudin, bivalirudin, or heparin.

7. The method of claim 1, further comprising, after step (vii), washing the tissue obtained in step (vii) with a solution containing an amino acid, followed by washing the tissue with a reducing agent.

8. The method of claim 1, wherein the solution used in each of steps (iv) to (vi) is 5 to 10 ml per 10cm² of the tissue.

9. The method of claim 1, wherein steps (iv) to (vi) are performed at 0 to 37°C.

10. The method of claim 1, wherein steps (iv) to (vi) are performed at 2 to 6°C.

11. The method of claim 1, wherein steps (iv) to (vi) are performed in a nitrogen atmosphere.

12. The method of claim 1, wherein steps (iv) to (vi) each are performed for 5 to 24 hours.

13. The method of claim 1, wherein the difunctional compound is a bis-epoxy compound.

14. The method of claim 1, wherein the solution containing the difunctional compound is a solution containing 0.5 to 2 mass% of polyethylene glycol-diglycidyl ether (PEG-DGE) or glutaraldehyde (GA).

15. The method of claim 1, wherein step (viii) is the step of exchanging, two to ten times, PBS in a volume ratio of 1000x or more or washing the tissue with PBS in the transmembrane liquid flow device.

16. The method of claim 1, further comprising sterilizing the tissue.

17. A decellularized tissue obtained by the method of any one of claims 1 to 16.
